# EUROPEAN PATENT APPLICATION

(11) **EP 4 310 487 A1**
(43) Date of publication of application: **24.01.2024**
(21) Application number: 21938554.9
(22) Date of filing: 30.04.2021
(51) Int. Cl.: G01N 27/00, C12M 1/34

(54) **PORE ARRAY LAYER STRUCTURE, CHIP DEVICE, NANOPORE SEQUENCING DEVICE, AND FILM FORMING METHOD AND USE THEREOF**

(71) Applicant: Qitan Technology Ltd., Chengdu, Sichuan 610041 (CN)
(72) Inventor: ZHANG, Zhe, Chengdu, Sichuan 610041 (CN); REN, Shilong, Chengdu, Sichuan 610041 (CN); SONG, Lu, Chengdu, Sichuan 610041 (CN)
(74) Representative: Boult Wade Tennant LLP
(86) International application number: PCT/CN2021/091732
(87) International publication number: WO 2022/227080

(57) **Abstract**

The present application discloses a pore array layered structure, a chip device, a nanopore sequencing device, a membrane forming method, and use thereof. The pore array layered structure is adapted for forming a membrane-forming space with a substrate, and the membrane-forming space is adapted to form a membrane layer. The pore array layered structure includes: a plurality of pore units arranged in an array, the pore units penetrating through the pore array layered structure and each including a first containing cavity and a second containing cavity communicated in an axial direction, first containing cavities are connected with the substrate, and at least one of the first containing cavities is communicated with other first containing cavities. The pore array layered structure provided by the present application can achieve serial communication and complementarity of the first non-polar medium among different first containing cavities and uniform distribution thereof, can ensure the uniformity of precoating so as to provide a better basis for membrane formation; during the stage of the membrane formation, the stability and serial communication and complementarity of the membrane layer can be ensured.

## Description

### TECHNICAL FIELD

The present application belongs to the field of biological detection technology, in particular to a pore array layered structure, a chip device, a nanopore sequencing device, a membrane forming method, and use thereof.

### BACKGROUND

Nanopore detection technology has advantages of high flux, high integration, parallelism, diversification and automation, and is widely used in fields such as protein detection, gene sequencing, and characterization of nanoparticles. For example, when conducting gene sequencing, a thin membrane of the gene sequencing device is provided with nanopores; when single-stranded deoxyribonucleic acid (DNA) molecules pass through the nanopores, different basic groups with different shape and size are subjected to specific reactions with detection molecules in the nanopores, causing changes in resistance. There is a constant voltage on two sides of the nanopores, and changes in the current when different basic groups pass through the nanopores can be detected, thereby reflecting the arrangement of basic groups in the DNA molecules passing through the nanopores.

The stability of the thin membrane of the gene sequencing device is crucial for the accuracy of detection, but currently, the stability of the thin membrane is poor, leading to poor detection accuracy.

### SUMMARY

The embodiments of the present application provide a pore array layered structure, a chip device, a nanopore sequencing device, a membrane forming method, and use thereof, which aim to solve the problem of poor stability of the thin membrane, and can improve the stability of membrane formation.

In a first embodiment, the embodiments of the present application provide a pore array layered structure for forming a membrane-forming space with a substrate, wherein the membrane-forming space is adapted to form a membrane layer. The pore array layered structure includes: a plurality of pore units arranged in an array, the pore units penetrating through the pore array layered structure and each including a first containing cavity and a second containing cavity communicated in an axial direction, the first containing cavities are connected with the substrate, and at least one of the first containing cavities is communicated with other first containing cavities.

According to embodiments in the first aspect, at least one of the second containing cavities is communicated with other second containing cavities.

According to any of the above embodiments in the first aspect, the pore array layered structure includes a first defining layer and a second defining layer, the first containing cavities are disposed in the first defining layer and penetrate through the first defining layer, and the second containing cavities are disposed in the second defining layer and penetrate through the second defining layer.

According to any of the above embodiments in the first aspect, the second defining layer includes a plurality of second sub units arranged in an array, and the second containing cavities are disposed in the second sub units and penetrate through the second sub units.

According to any of the above embodiments in the first aspect, the second defining layer includes a plurality of third sub units arranged in an array, the third sub units include third sub containing cavities, and the second sub units are located in the third sub containing cavities with gaps being formed between the second sub units and the third sub units.

According to any of the above embodiments in the first aspect, a plurality of first grooves are provided on outer peripheral sides of respective second sub units.

According to any of the above embodiments in the first aspect, the first defining layer includes at least one first channel, by which at least two adjacent first containing cavities are communicated with each other.

According to any of the above embodiments in the first aspect, the second defining layer includes at least one second channel, by which at least two adjacent second containing cavities are communicated with each other.

According to any of the above embodiments in the first aspect, the first defining layer includes at least one first communicating groove, the first communicating groove extends outward from the first containing cavity in a radial direction, and an end of the first communicating groove facing away from the first containing cavity is closed.

According to any of the above embodiments in the first aspect, the first defining layer includes at least one first communicating groove, and the first communicating groove penetrates through the first defining layer.

According to any of the above embodiments in the first aspect, the second defining layer includes at least one second communicating groove, the second communicating groove extends outward from the second containing cavity in a radial direction, and an end of the second communicating groove facing away from the second containing cavity is closed.

According to any of the above embodiments in the first aspect, the second defining layer includes at least one second communicating groove, and the second communicating groove penetrates through the second defining layer.

According to any of the above embodiments in the first aspect, the first defining layer includes at least one first communicating groove, the at least one first communicating groove forms a third containing cavity, and the third containing cavity has a diameter or width greater than that of the second containing cavity.

According to any of the above embodiments in the first aspect, the first containing cavity has a diameter or width less than or equal to that of the second containing cavity.

In a second aspect, the embodiments of the present application provide a chip device, including a substrate; the pore array layered structure of the above embodiments in the first aspect, the pore array layered structure is located on the substrate, the pore array layered structure includes a plurality of pore units arranged in an array, and second containing cavities of the pore units are located on a side of the first containing cavities of the pore units opposite to the substrate; and membrane layers located in the plurality of pore units, wherein the membrane layers are configured to test samples to be tested.

In a third aspect, the embodiments of the present application provide a membrane forming method, including: disposing a first non-polar medium in the pore array layered structure and forming a precoating layer on a surface of the pore array layered structure; flowing a first polar medium through the pore array layered structure to replace at least part of the first non-polar medium; flowing a second non-polar medium through the pore array layered structure to replace at least part of the first polar medium, wherein the second non-polar medium includes amphiphilic molecular materials; and flowing a second polar medium through the pore array layered structure to replace at least part of the second non-polar medium and to form membrane layers at interface between the first polar medium and the second polar medium, wherein the membrane layers contain the amphiphilic molecular materials.

In a fourth aspect, the embodiments of the present application provide a nanopore sequencing device, including the pore array layered structure of the embodiments in the first aspect of the present application, the chip device of the embodiments in the second aspect of the present application, or the membrane layers prepared by the membrane forming method of the embodiments in the third aspect of the present application.

A fifth aspect is use of the pore array layered structure of the embodiments in the first aspect of the present application, the chip device of the embodiments in the second aspect of the present application or the membrane layers prepared by the membrane forming method of the embodiments in the third aspect of the present application in characterizing an analyte, wherein the analyte includes biological polymer, and the biological polymer is selected from one of polynucleotide, polypeptide, polyose and lipide.

According to any of the above embodiments in the fifth aspect of the present application, the biological polymer is polynucleotide, which includes DNA and/or RNA.

According to the embodiments of the present application, the pore array layered structure includes a plurality of pore units arranged in the array, and membrane layers are formed within the pore units; each pore unit includes a first and second containing cavities communicated with each other, at least one of the first containing cavities is communicated with other first containing cavities, and the first non-polar medium located in the first containing cavities can diffuse and flow between adjacent first containing cavities, achieving serial communication and complementarity of the first non-polar medium among different first containing cavities and uniform distribution thereof; on the one hand, it can ensure the uniformity of precoating so as to provide a better basis for membrane formation; on the other hand, during the stage of the membrane formation, the stability and serial communication and complementarity of the membrane layers can be ensured.

### BRIEF DESCRIPTION OF THE DRAWINGS

The features, advantages and technical effects of the exemplary embodiments of the present application will be described below with reference to the accompanying drawings.
Fig. 1 is a perspective view of a pore array layered structure provided according to an embodiment of the present application;
Fig. 2 is a schematic diagram of membrane formation;
Fig. 3 is an explosive perspective view of a pore position defining layer provided according to an embodiment of the present application;
Fig. 4 is a top view of a pore position defining layer provided according to an embodiment of the present application;
Fig. 5 is an explosive perspective view of a pore position defining layer provided according to a first alternative embodiment of the present application;
Fig. 6 is a top view of the pore position defining layer provided according to the first alternative embodiment of the present application;
Fig. 7 is a top view of a second defining layer provided according to the first alternative embodiment of the present application;
Fig. 8 is a perspective view of a pore position defining layer provided according to a second alternative embodiment of the present application;
Fig. 9 is an explosive perspective view of the pore position defining layer provided according to the second alternative embodiment of the present application;
Fig. 10 is a top view of the pore position defining layer provided according to the second alternative embodiment of the present application;
Fig. 11 is an explosive perspective view of a pore position defining layer provided according to a third alternative embodiment of the present application;
Fig. 12 is a top view of the pore position defining layer provided according to the third alternative embodiment of the present application;
Fig. 13 is a first top view of a second defining layer provided according to the third alternative embodiment of the present application;
Fig. 14 is a second top view of the second defining layer provided according to the third alternative embodiment of the present application;
Fig. 15 is a third top view of the second defining layer provided according to the third alternative embodiment of the present application;
Fig. 16 is a an explosive perspective view of a pore position defining layer provided according to a fourth alternative embodiment of the present application;
Fig. 17 is a top view of the pore position defining layer provided according to the fourth alternative embodiment of the present application;
Fig. 18 is a top view of a second defining layer provided according to the fourth alternative embodiment of the present application;
Fig. 19 is a schematic diagram of section A-A of Fig. 18;
Fig. 20 is a top view of a second defining layer provided according to a fifth alternative embodiment of the present application;
Fig. 21 is a structural schematic diagram of a chip device provided according to an embodiment of the present application;
Fig. 22 shows a principle diagram of electrical characterization test;
Fig. 23 is an electrical characterization diagram of a background of the chip device before membrane formation provided according to an embodiment of the present application;
Fig. 24 is an electrical characterization diagram of a chip device of a comparative example;
Fig. 25 is an electrical characterization diagram of a chip device provided according to an embodiment of the present application;
Fig. 26 is a flow chart of a membrane forming process provided according to an embodiment of the present application;
Fig. 27 is a structural schematic diagram of a membrane layer.

### BEST EMBODIMENTS OF THE PRESENT DISCLOSURE

The implementations of the present application will be further described in detail in combination with the accompanying drawings and embodiments. The following detailed description of the embodiments and the accompanying drawings are used to illustrate the principles of the present application by way of example, but cannot be intended to limit the scope of the present application, that is, the present application is not limited to the described embodiments.

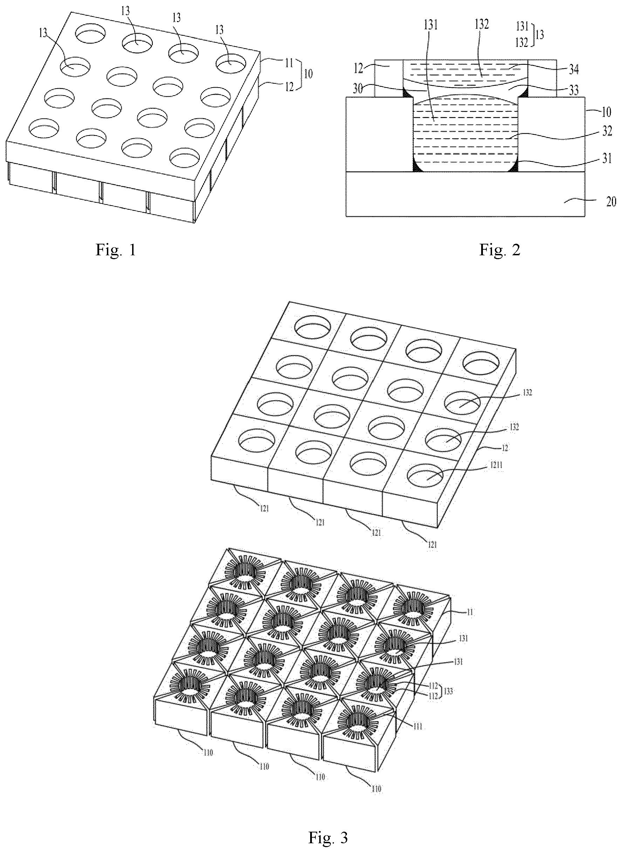

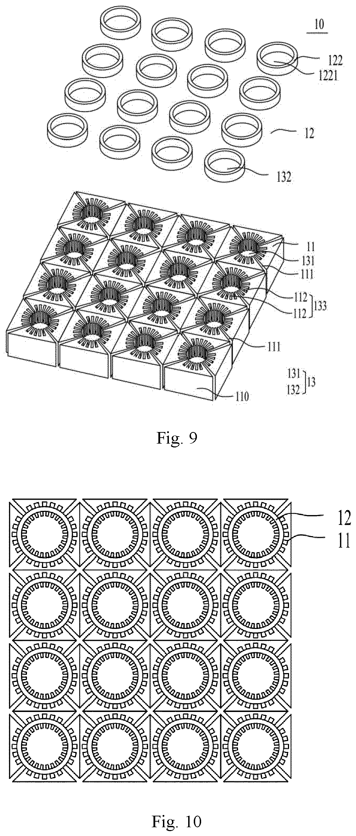

In order to better understand the present application, the embodiments of the present application are described below in combination with Figs. 1 to 27. In a first aspect, the embodiments of the present application provide a pore array layered structure. Fig. 1 is a structural schematic diagram of a pore array layered structure provided by an embodiment of the present application. Fig. 2 is a schematic diagram of membrane formation provided by an embodiment of the present application. As shown in Fig. 1 and Fig. 2, the pore array layered structure 10 provided by the embodiments of the present application may be adapted to form a membrane-forming space with a substrate 20, and the membrane-forming space is adapted to form a membrane layer 30. The pore array layered structure 10 includes a plurality of pore units 13 arranged in an array, and each pore unit 13 penetrates through the pore array layered structure 10. Each pore unit 13 includes a first containing cavity 131 and a second containing cavity 132 communicated in an axial direction. The first containing cavities 131 are configured to connect with the substrate 20. The first containing cavities 131 penetrate through a side portion of the pore array layered structure 10 close to the substrate 20, the second containing cavities 132 penetrate through a side portion of the pore array layered structure 10 facing away from the substrate 20, and at least one of the first containing cavities 131 is communicated with other first containing cavities 131.

As shown in Fig. 2, in the pore array layered structure 10 according to the embodiments of the present application, the membrane layer 30 is formed within the pore unit 13. Specifically, a membrane forming method may include step S200, step 300, step 400 and step 500.

In the step S200, a first non-polar medium 31 is disposed in the pore array layered structure 10, and a precoating layer is formed on a surface of the pore array layered structure 10.

In the step S300, a first polar medium 32 is flowed through the pore array layered structure 10 to replace at least part of the first non-polar medium 31.

In the step S400, a second non-polar medium 33 is flowed through the pore array layered structure 10 to replace at least part of the first polar medium 32, wherein the second non-polar medium 33 contains amphiphilic molecular materials.

In the step S500, a second polar medium 34 is flowed through the pore array layered structure 10 to replace at least part of the second non-polar medium 33 and form the membrane layer 30 at interface between the first polar medium 32 and the second polar medium 34, wherein the membrane layer 30 contains the amphiphilic molecular materials.

According to the pore array layered structure 10 of the embodiments of the present application, the pore array layered structure 10 includes a plurality of pore units 13 arranged in an array, and the membrane layer 30 is formed in each pore unit 13; each of the pore units 13 includes a first containing cavity 131 and a second containing cavity 132 communicated with each other, at least one of the first containing cavities 131 is communicated with other first containing cavities 131, a first non-polar medium 31 located in the first containing cavity 131 can diffuse and flow between adjacent first containing cavities 131 and form a precoating layer on a surface of the pore unit 13, realizing serial communication and complementarity of the first non-polar medium 31 among different first containing cavities 131 and uniform distribution thereof; on the one hand, the uniformity of precoating can be ensured to provide a better basis for membrane formation. On the other hand, the solvent of the membrane layer 30 is the second non-polar medium 33, the second non-polar medium 33 and the first non-polar medium 31 have good compatibility, and thus the membrane layer 30 and the precoating layer have good compatibility; at the stage of membrane formation, the membrane layer 30 can flow and complement with the membrane layer 30 in adjacent first containing cavity 131 through the precoating layer, and this can ensure stability, serial communication and complementarity of the membrane layer 30.

As shown in Fig. 2, in some embodiments, at least one of the second containing cavities 132 is communicated with other second containing cavities 132. The first non-polar medium 31 can diffuse and flow between adjacent second containing cavities 132, realizing serial communication and complementarity of the first non-polar medium 31 among different second containing cavities 132, and further ensuring the stability and serial complementarity of the membrane layer 30.

When the membrane layer 30 is located at the first containing cavity 131, at the stage of membrane formation, the membrane layer 30 can flow and complement with the membrane layer 30 in adjacent first containing cavity 131 through the precoating layer located at the first containing cavities 131, so as to ensure uniform distribution of the membrane layer 30. When the membrane layer 30 is located at the second containing cavity 132, at the stage of membrane formation, the membrane layer 30 can flow and complement with the membrane layer 30 in adjacent second containing cavity 132 through the precoating layer located at the second containing cavities 132, so as to ensure the uniform distribution of the membrane layer 30.

Certainly, adjacent second containing cavities 132 may not be communicated with each other. In this case, volatilization or mutual dissolution of mediums outside the membrane forming region can be prevented. Moreover, when the membrane layer 30 is located at the second containing cavity 132, at the stage of membrane formation, the membrane layer 30 can pass through the precoating layer located in the second containing cavity 132 and the first containing cavity 131 in order and flow into the first containing cavity 131, so as to realize the flow complementarity with the membrane layer in adjacent pore unit 13.

As shown in Fig. 3, in some embodiments, a pore position defining layer 10 may include a first defining layer 11 and a second defining layer 12. When assembled with the substrate 20, the first defining layer 11 may be disposed on the substrate 20, and the first containing cavities 131 are disposed on the first defining layer 11. The first defining layer 11 may include a plurality of first sub units 110 arranged in an array. The first containing cavities 131 are disposed in the first sub units 110.

As shown in Fig. 3, in some embodiments, the first defining layer 11 may include one or more first channels 111, by which at least two adjacent first containing cavities 131 are communicated with each other. The adjacent first containing cavities 131 may be adjacent in a longitudinal direction, a transverse direction or a diagonal direction. The serial communication and complementarity of the first non-polar medium 31 in adjacent first containing cavities 131 can be realized through the first channel 111. When the first channel 111 are multiple, the first non-polar medium 31 can flow in more channels, a rapid serial communication and complementarity of the first non-polar medium 31 and/or the membrane layer 30 can be achieved, and a membrane formation rate can be improved.

As an example, the first channels 111 may be first slots provided in the first defining layer 11. The first slots may be provided in one single direction or in multiple directions. For example, the first slots may be provided in radial directions or diagonal directions of the first sub units 110, and can communicate two first containing cavities 131 adjacent in the diagonal direction with each other, or even communicate four first containing cavities 131 adjacent in the diagonal direction, transverse direction and longitudinal direction with each other, so as to realize rapid serial communication and complementarity of the first non-polar medium 31, realize serial communication and complementarity in more channels, and improve the membrane formation rate. For another example, the first slots may be provided in the transverse direction or longitudinal direction of the first sub units 110, and the first slots may communicate the first containing cavities 131 adjacent in the transverse direction or the longitudinal direction with each other. For another example, the first slots may be provided in both the radial and transverse directions of the first sub units 110, and the first slots may communicate the first containing cavities 131 adjacent in the transverse and longitudinal directions with each other.

As another example, the first channel 111 may be a connecting tube included in the first defining layer 11, and the connecting tube may be embedded in the first defining layer 11. The specific structure of the first channel 111 is not limited here.

Optionally, groove structures may further be provided on an inner side of the first channel 111, and the first non-polar medium 31 can occupy spaces in the groove structures, which is conducive to the serial communication and complementarity of the first non-polar medium 31.

With the increase of flow cross-sections of the first channels 111, a flow volume of the first non-polar medium 31 increases, and the speed of serial communication and complementarity of the first non-polar medium 31 and/or the membrane layer 30 can also be increased. For example, the first slots may penetrate through the first defining layer 11, and the flow cross-sections of the first slots are relatively large.

In some embodiments, the first defining layer 11 may further include at least one first communicating groove 112, which is communicated with the first containing cavity 131 and extends outward from the first containing cavity 131 in a radial direction, and an end of the first communicating groove 112 facing away from the first containing cavity 131 is closed. The at least one first communicating groove 112 can form a channel structure extending from the first containing cavity 131, and the first communicating groove 112 can form a capillary structure of the first sub unit 110; during a preocating process, the first non-polar medium 31 can stably occupy the space in the capillary structure, and prevent the subsequent first polar medium 32 from entering the first communicating groove 112, so as to facilitate the serial communication and complementarity of the first non-polar medium 31 to adjacent first containing cavity 131, and thus ensure the serial communication and complementarity, and stability of the membrane layer 30.

Alternatively, the first communicating groove 112 may penetrate through the first defining layer 11. The first communicating groove 112 has a relatively large flow cross-section, and serial communication and complementarity of the first non-polar medium 31 in the first communicating groove 112 can be performed in a larger range.

As an example, the first communicating groove 112 may be provided in the radial or diagonal direction of the first sub unit 110. Certainly, a plurality of first communicating grooves 112 may be provided and uniformly distributed in a circumferential direction of the first sub unit 110. The plurality of first communicating grooves 112 form a plurality of capillary structures, thereby increasing channels for flowing of the first non-polar medium 31.

According to the embodiments of the present application, when the pore array layered structure 10 and the substrate 20 are assembled into an integrity, the second defining layer 12 may be located on a side of the first defining layer 11 facing away from the substrate 20, and the second containing cavities 132 are disposed in the second defining layer 12. The second defining layer 12 may serve as an auxiliary structure for stabilizing the membrane conformation.

In some embodiments, the second defining layer 12 may be formed into an interconnected structure or discrete structures. The second defining layer 12 can support the membrane layer 30.

As shown in Fig. 3 to Fig. 7, as an example, the second defining layer 12 may be formed into third sub units 121 such as cover plates, distributed in an array, the cover plates are interconnected, and each third sub unit 121 (such as the cover plate) may include a third sub containing cavity 1211. The interconnected cover plates form the second defining layer 12. The second defining layer 12 formed into the interconnected structure, can prevent mutual dissolution or volatilization of the first polar medium 32 and the first non-polar medium 31 in the region outside the pore units 13, which is conducive to relative separation of the non-polar medium and the polar medium at the non-membrane forming position, and brings the advantage of storage timeliness. The third sub units 121 distributed in an array may be integrally formed, to facilitate the processing and manufacturing. In this case, the third sub containing cavity 1211 may correspond to the second containing cavity 132, and form the pore unit 13 with the first containing cavity 131.

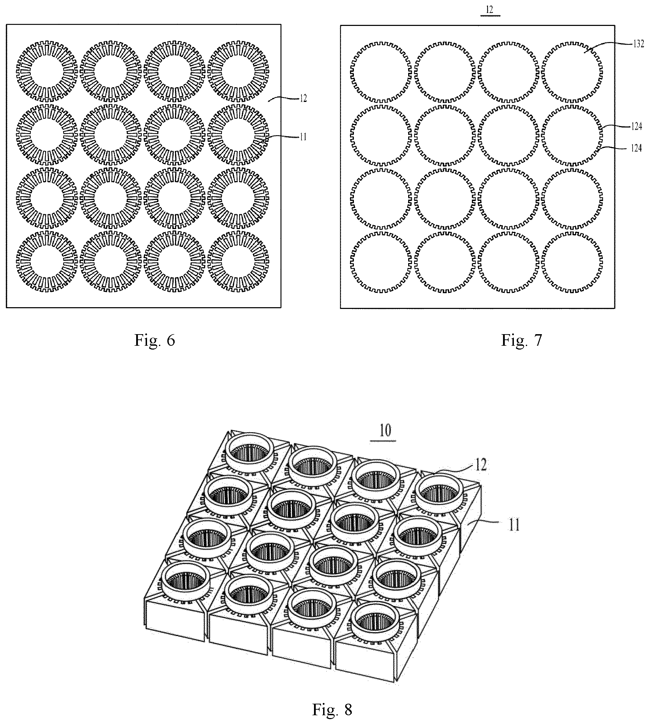

As shown in Fig. 8 to Fig. 15, as another example, the second defining layer 12 may include a plurality of second sub units 122 such as column bodies, distributed in an array, with gaps formed between adjacent column bodies, each column body is an independent structure, and each column body includes a second sub containing cavity 1221 in it. The second defining layer 12 is formed as discrete structures. The column body may be formed in a shape of a cylindrical column, a multilateral column, and the like, which is not limited here. The medium in adjacent column bodies can be communicated with each other, which is conducive to the mutual serial communication and complementarity of the first non-polar medium 31, and can improve stability of the membrane layer 30. In this case, the second sub containing cavity 1221 can correspond to the second containing cavity 132, and forms the pore unit 13 with the first containing cavity 131.

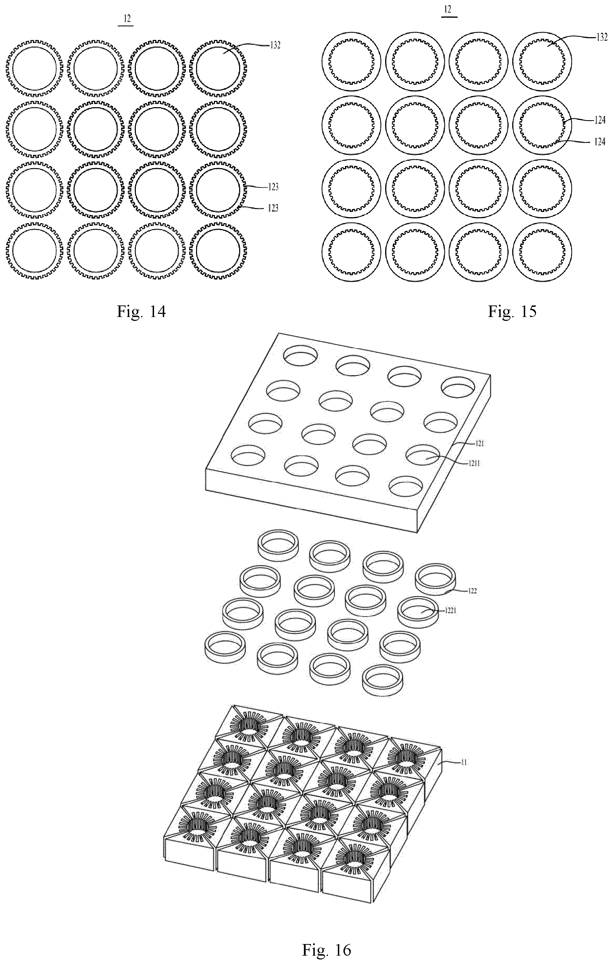

As shown in Fig. 16 to Fig. 19, as another example, the second defining layer 12 may include a plurality of third sub units 121 distributed in an array and a plurality of second sub units 122 distributed in an array. Each third sub unit 121 includes a third sub containing cavity 1211. Each second sub unit 122 includes a second sub containing cavity 1221, the second sub unit 122 is located in the third sub containing cavity 1211, and the second sub containing cavity 1221 can serve as the second containing cavity 132 to form the pore unit 13 with the first containing cavity 131. The third sub containing cavity 1211 of the third sub unit 121 provides a placing region for the second sub containing cavity 1221 of the second sub unit 122. Moreover, other regions among the plurality of third sub units 121 are continuous, which can prevent the mutual dissolution or volatilization of the polar medium and the non-polar medium at the region outside the pore units 13. For example, the third sub unit 121 includes a cover plate, which includes the third sub containing cavity 1211; the second sub unit 121 includes a column body, in which the second containing cavity 132 is disposed, and each column body is located in the corresponding third sub containing cavity 1211 of the cover plate.

According to the embodiments of the present application, a gap may be formed between the third sub unit 121 and the second sub unit 122 to control the morphology of the membrane layer 30 during the membrane formation process.

As shown in Fig. 16 to Fig. 19, as an example, the third sub unit 121 is formed as a cover plate, and the second sub unit 122 is formed as a cylindrical body; the cylindrical body is located within the cover plate, and there is a gap between the cylindrical body and the cover plate; the cylindrical body is more conducive to formation of the interface morphology of the first polar medium 32 and the first non-polar medium 31 during the membrane formation process.

In particular, when the first defining layer 11 includes at least one first communicating groove 112, which forms the capillary structure, the cylindrical body is easier to form, with the capillary structure, a structural model including the interface between the first polar medium 32 and the first non-polar medium 31.

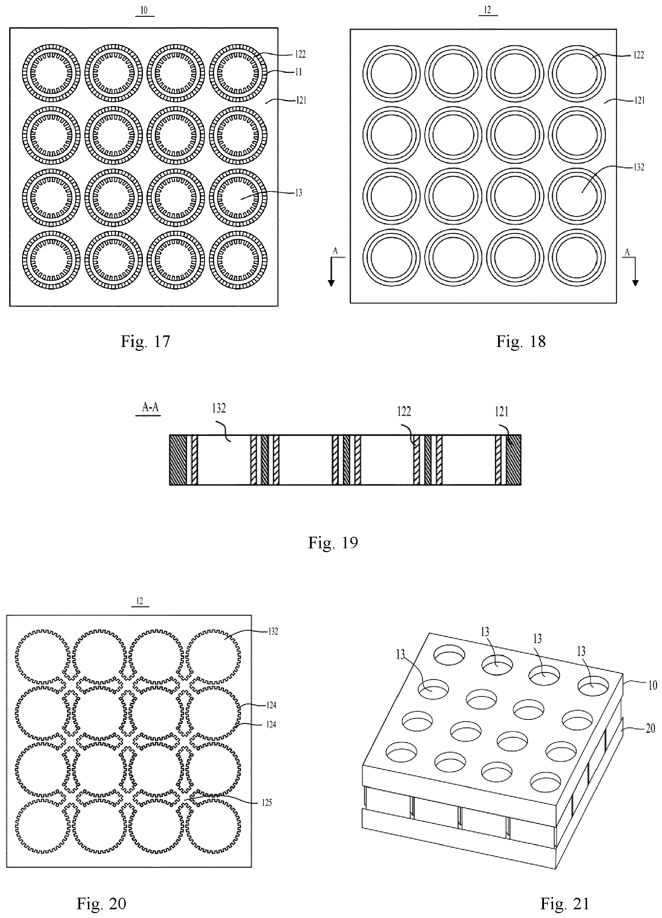

As shown in Fig. 20, in some embodiments, the second defining layer 12 may include one or more second channels 125, by which at least two adjacent second containing cavities 132 are communicated with each other. The at least two adjacent second containing cavities 132 may be adjacent in a longitudinal direction, a transverse direction or a diagonal direction. The first non-polar medium 31 in adjacent second containing cavities 132 can be serially communicated and complemented each other through the second channel 125.

Alternatively, the second channel 125 may further be provided with groove structures on its inner side to facilitate space occupation of the first non-polar medium 31. It should be noted that the specific examples and functions of the second channel 125 are basically the same as those of the first channel 111, and will not be repeated here.

In some embodiments, at least one first groove 123 is provided on an outer peripheral side of the second sub unit 122 of the second defining layer 12, and the first groove 123 can realize the serial communication and complementarity of the first non-polar medium 31 in the second defining layer 12.

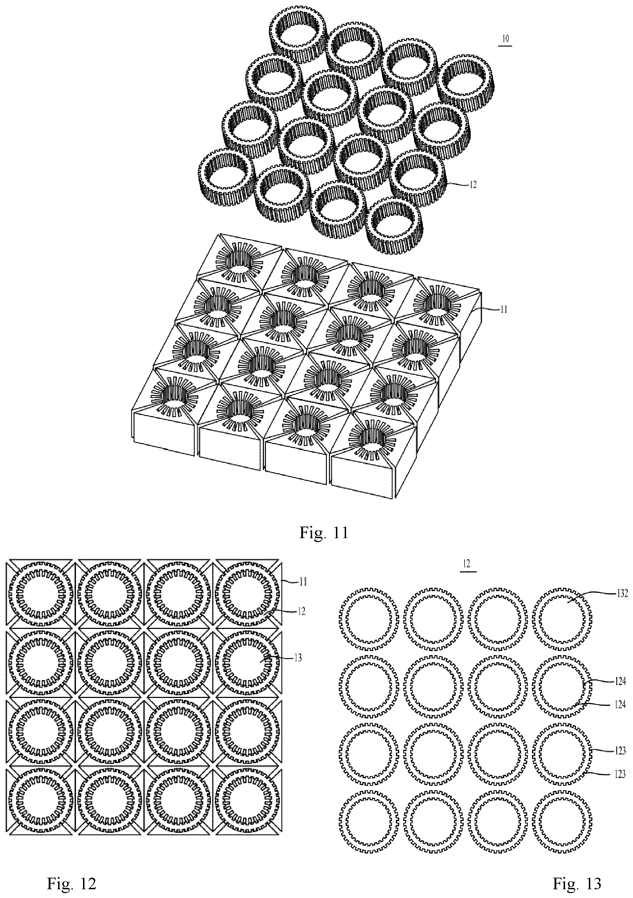

As shown in Figs. 11-14, as an example, the first groove 123 may be provided on an outer peripheral side of the second sub unit 122 extending in a radial or diagonal direction. For example, the second sub unit 122 includes a column body, a plurality of column bodies distributed in an array form the second defining layer 12, and at least one first groove 123 is provided on an outer peripheral side of each column body. Each of the first grooves 123 can be used for space occupation of the first non-polar medium 31, so that serial communication and complementarity of the first non-polar medium 31 can achieved between adjacent column bodies.

In some embodiments, the second defining layer 12 may further include at least one second communicating groove 124, which is communicated with the second containing cavity 132, the second communicating groove 124 extends outward from the second containing cavity 132 in a radial direction, and an end of the second communicating groove 124 facing away from the second containing cavity 132 is closed. At least one second communicating groove 124 can form a channel structure extending from the second containing cavity 132, and during the precoating process, the first non-polar medium 31 can occupy space in the second communicating groove 124 prior to the first polar medium 32 and prevent the first polar medium 32 from entering the second communicating groove 124, so as to facilitate the serial communication and complementarity of the first non-polar medium 31 to the adjacent second containing cavity 132, thereby ensuring the serial communication and complementarity, and stability of the membrane layer 30. It should be noted that the specific examples and functions of the second communicating groove 124 are the same as those of the first communicating groove 112, and will not be repeated here.

As shown in Fig. 13, as an example, the second defining layer 12 includes a plurality of column bodies distributed in an array, and each column body is provided with at least one second communicating groove 124 on its inner side. Each column body forms the second sub unit 122. Each second communicating groove 124 can be used for space occupation of the first non-polar medium 31, so that serial communication and complementarity of the first non-polar medium 31 can be realized between adjacent column bodies.

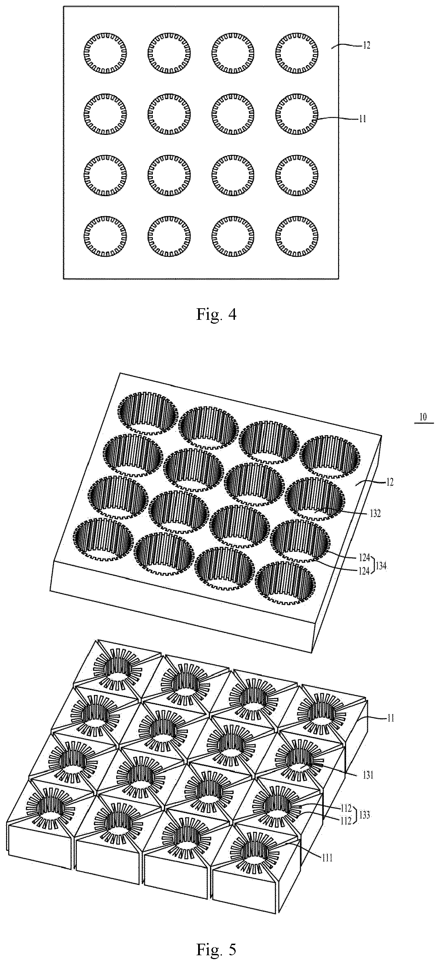

As shown in Figs. 5 to 7, as another example, the second defining layer 12 includes a plurality of cover plates distributed in an array, each cover plate forms one third sub unit 121, and at least one second communicating groove 124 is provided in the cover plate. Each second communicating groove 124 can be used for space occupation of the first non-polar medium 31, so that serial communication and complementarity of the first non-polar medium 31 can be realized between adjacent second containing cavities 132.

As another example, the second defining layer 12 includes a plurality of cover plates distributed in an array and a plurality of column bodies distributed in an array. Each cover plate forms one third sub unit 121, and each column body forms one second sub unit 122. At least one of the cover plate and the column body is provided with at least one second communicating groove 124 on its inner side; for example, the inner side of the cover plate is provided with the second communicating groove 124; for example, the inner side of the column body is provided with the second communicating groove 124; for example, the inner side of the cover plate and the inner side of the column body are each provided with the second communicating groove 124. The specific function of the second communicating groove 124 is consistent with that in the above example, and will not be repeated here.

According to the embodiments of the present application, the first containing cavity 131 has a diameter or width smaller than or equal to that of the second containing cavity 132, and in this state, the second defining layer 12 can provide an auxiliary structure for the first defining layer 11 to stable the membranes.

In some embodiments, the at least one first communicating groove 112 included in the first defining layer 11 forms a third containing cavity 133, which may have a diameter or width larger than or equal to that of the second containing cavity 132.

Taking the diameter or width of the first containing cavity 131 as a reference, when the diameter or width of the second containing cavity 132 is larger than that of the first containing cavity 131, with the decrease of the diameter or width of the second containing cavity 132, it is more conducive to form a membrane layer 30 with a smaller size, and the state of the membrane layer 30 is more stable. The diameter or width of the second containing cavity 132 may be decreased to be equal to that of the first containing cavity 131. However, when the diameter or width of the second containing cavity 132 is further reduced, an opening of the second containing cavity 132 is small, which is not conducive to a sample to be tested entering the pore unit 13 for test.

When the diameter or width of the second containing cavity 132 is smaller than or equal to that of the third containing cavity 133, with the increase of the diameter or width of the second containing cavity 132, the second defining layer 12 can still provide an auxiliary structure for the first defining layer 11 to stable the membranes.

According to the embodiment of the present application, a height of the first containing cavity 131 of the first defining layer 11 is greater than the width or diameter of the first containing cavity 131; for example, a ratio of the height of the first containing cavity 131 to the width or diameter of the first containing cavity 131 may be 0.8 to 3:1, and the specific ratio may be 0.8:1, 0.9:1, 1.0:1, 1.1:1, 1.2:1, 1.3:1, 1.4:1, 1.5:1, 1.8:1, 2.0:1, 2.5:1 or 3:1. The specific range of the ratio may be a range composed of any two of above values. Within the range defined by the above values, the first containing cavity 131 has a high aspect ratio, which is conducive to the diffusion and space occupation of the first non-polar medium 31 and improves the stability of membrane formation; moreover, the membrane layer 30 has uniform thickness and better functionality.

According to the embodiments of the present application, at least one of the second sub unit 122 and the third sub unit 121, together with the first sub unit 110 can form one structural unit of the pore array layered structure 10, and such structural unit generally has a width less than 1000 µm. The pore array layered structure 10 occupies a small space and can contain a large number of structural units, and thus can test a larger number of samples.

In the case that a ratio of the height of the first containing cavity 131 to a width of the first communicating groove 112 is more than 3, or a ratio of a height of the second containing cavity 132 to a width of the second communicating groove 124 is more than 3, the first communicating groove 112 or the second communicating groove 124 can form the capillary structure with appropriate size to facilitate the space occupation of the first non-polar medium 31.

In a second aspect, the embodiments of the present application provide a chip device. Fig. 21 is a structural schematic diagram of a chip device provided according to an embodiment of the present application. As shown in Fig. 21, the chip device provided by the embodiment of the present application may include a substrate 20, the pore array layered structure 10 according to any of the above embodiments and membrane layers 30. The pore array layered structure 10 is disposed on the substrate 20; the membrane layers 30 are located in the plurality of pore units 13 distributed in an array in the pore position defining layer 10, and the membrane layers 30 are configured to test samples to be tested.

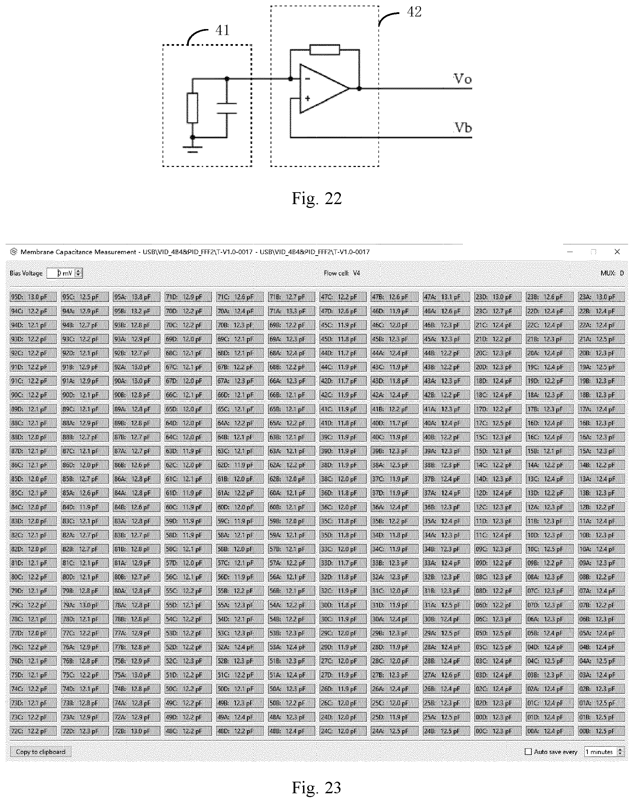

Fig. 22 shows a principle diagram of an electrical characterization detection; as shown in Fig. 22, the chip device is electrically characterized by detecting with an electrical characterization device. The electrical characterization device may be composed of a multi-channel current detection circuit 42 with adjustable bias voltage, and the current detection circuit 42 is electrically connected with an object under detection, such as a chip device 41, to detect performance of the chip device 41. The current detection circuit 42 may be a trans-impedance amplifier (TIA), a capacitance trans-impedance amplifier (CTIA), or in other forms. Taking the trans-impedance amplifier as an example, working principle of the electrical characterization is as follows:
applying a bias voltage Vb to an in-phase input (+) of an operational amplifier. Due to existence of a negative feedback circuit, the operational amplifier will adjust an output voltage Vo, so that a voltage at its inverting input (-) is the same as that at its in-phase input, that is, the bias voltage Vb is applied to the object under detection.

Because the operational amplifier has a very large input impedance, it can be considered that there is no current flowing into or out of the operational amplifier. Therefore, a current flowing through the object under detection will all pass through a feedback resistance of the trans-impedance amplifier and is reflected in the form of voltage at the output of the operational amplifier. If the feedback resistance is Rf, the current on the object under detection I=(Vo-Vb)/Rf. The DC resistance of the object under detection can be obtained by dividing the bias voltage Vb by the current I detected by the current detection circuit. To detect a capacitance of the object under detection, it is only necessary to apply a specific AC signal (such as sine wave, triangular wave, etc.) to the object under detection and detect its current response, and thus the capacitance of the object under detection can be calculated.

Shade of displayed color of the electrical characterization in each unit in an instrument is positively correlated with a value of a membrane capacitance, that is, the darker the color, the greater the value of membrane capacitance. When the value is less than 20 pf, it is a background capacitance value of the instrument or a capacitance value of an initial state when the membrane is not formed, which is displayed in light gray; 20.1-30 pf is the membrane capacitance value that is not conducive to subsequent conventional pore embedding of the membrane layer containing amphiphilic molecular materials, which is displayed in gray; 30.1-65 pf is the membrane capacitance value suitable for subsequent conventional pore embedding of the membrane layer containing amphiphilic molecular materials, which is displayed in dark gray; the value more than 65 pf implies a broken membrane or that the membrane layer containing amphiphilic molecular materials does not have the ability of being embedded in the pores, which is displayed in black.

Fig. 23 shows an electrical characterization diagram of a background of a chip device before the membrane is formed. As shown in Fig. 23, since the first non-polar medium 31 of the first defining layer 11 and the second non-polar medium 33 of the second defining layer 12 are isolated by the second polar medium 32 interposed between them, the capacitance in this case only represents the background value of a circuit system of the instrument itself.

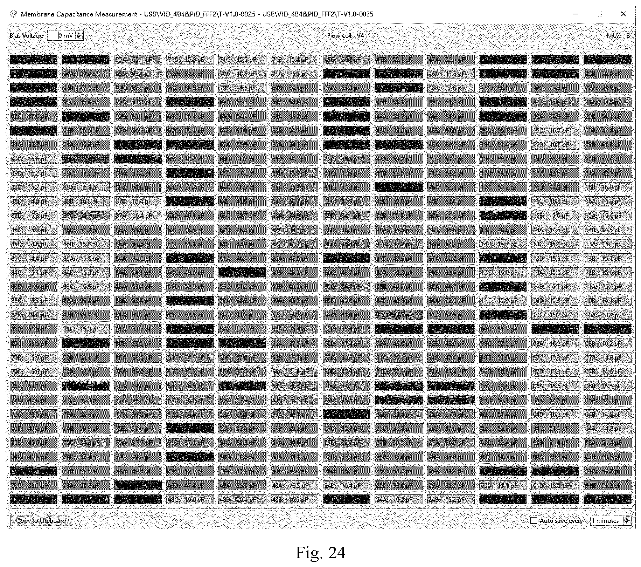

Fig. 24 shows an electrical characterization diagram of a chip device of a comparative example. In the chip device of the comparative example, adjacent first containing cavities 131 are communicated with each other while adjacent second containing cavities 131 of the chip device are not communicated. As shown in Fig. 24, the capacitance value after the membrane being formed gradually increases compared to that before the membrane being formed, and 95% of the capacitance values are in a range of 45-55 pf.

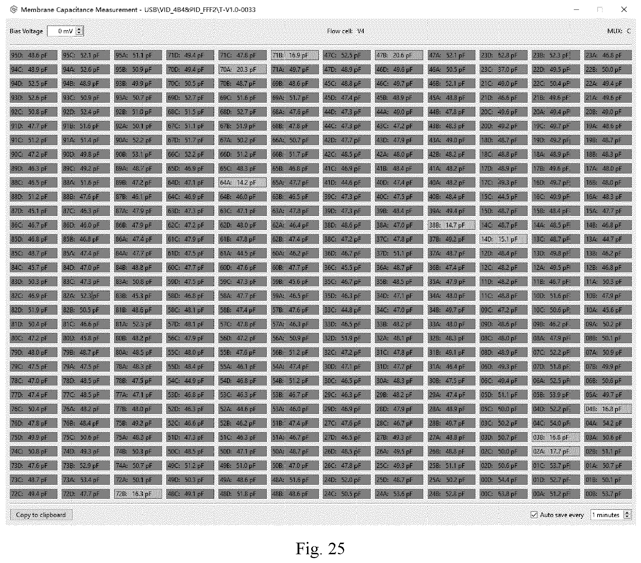

Fig. 25 shows an electrical characterization diagram of a chip device according to an embodiment of the present application. As shown in Fig. 25, the membrane layers 30 have uniform thickness and are relatively thin.

The chip device according to the embodiments of the present application can realize the serial communication and complementarity of the first non-polar medium 31 among different first containing cavities 131 and uniform distribution thereof, and thus can improve the stability of membrane formation.

In a third aspect, the embodiments of the present application provide a membrane forming method, including step S 100 to step S500.

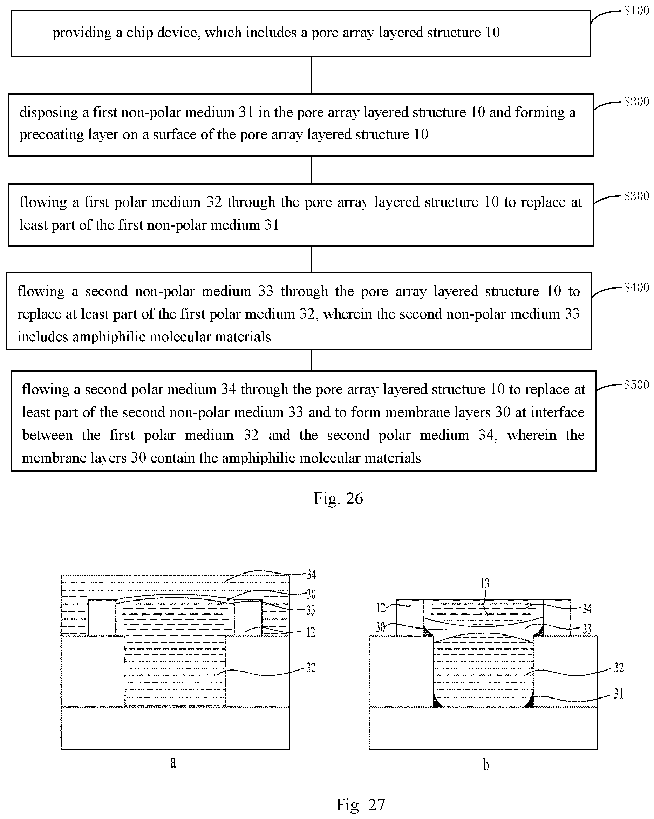

Fig. 26 shows a schematic diagram of a membrane forming process provided by an embodiment of the present application. As shown in Fig. 26, in the step S100, a chip device is provided, which includes a pore array layered structure 10. The chip device can be the chip device of the embodiments in the second aspect of the present application;

In step S200, the first non-polar medium 31 is disposed in the pore array layered structure 10, to form a precoating layer on a surface of the pore array layered structure 10. The pore units 13 are precoated with the first non-polar medium 31.

The first non-polar medium 31 can be coated on surfaces of the pore units 13 and thus occupy the surfaces of the pore units 13; in the subsequent driving step, the first non-polar medium 31 still occupies positions of the surfaces of the pore units 13. The first non-polar medium 31 in different first containing cavities 131 can flow and complement each other, and thus the first non-polar medium 31 can be uniformly distributed; when the membrane layers 30 are formed, complementarity of the membrane layers 30 in adjacent pore units 13 can be realized through the precoating layer to ensure the stability of the membrane layers 30.

In step S300, a first polar medium 32 is flowed through the pore array layered structure 10 to replace at least part of the first non-polar medium 31. The first polar medium 32 partially drives away the first non-polar medium 31.

In step S400, a second non-polar medium 33 is flowed through the pore array layered structure 10 to replace at least part of the first polar medium 32, wherein the second non-polar medium 33 contains amphiphilic molecular materials. The first polar medium 32 in the pore units 13 is partially driven away by the second non-polar medium 33.

It should be noted that for the first non-polar medium 31 and the second non-polar medium 33, different kinds of substances can be selected, and certainly, the same kind of substances can be selected, but the second non-polar medium 33 can contain amphiphilic molecular materials. The amphiphilic molecular materials are dissolved in the second non-polar medium 33, and refer to materials with both hydrophilicity and lipophilicity; as an example, the amphiphilic molecular materials can be phospholipids, block copolymers, etc. For example, 1,2-diphytanoyl-sn-glycero-3-phosphocholine (DPhPC), Distearoyl phosphoethanolamine (DSPE), Diblock Copolymer (DBCP), Tri-block Copolymer (TBCP), etc.

In step S500, a second polar medium 34 is flowed through the pore array layered structure 10 to replace at least part of the second non-polar medium 33 and form membrane layers 30 at interface between the first polar medium 32 and the second polar medium 34, wherein the membrane layers 30 contain the amphiphilic molecular materials. It should be noted that for the first polar medium 32 and the second polar medium 34, different kinds of substances can be selected, and certainly, the same kind of substances can be selected; there is no specific limitation here.

Referring to Fig. 27, where Fig. 27a is a schematic diagram of membrane formation of a comparative example, and Fig. 27b is a schematic diagram of membrane formation in an embodiment of the present application. As shown in Fig. 27a, the comparative example does not include the step S200, that is, there is no precoating process for forming a layer, and the first polar medium 32 is directly filled to the pore array layered structure 10.

Although the pore array layered structure 10 can be made of non-polar materials with lipophilic and hydrophobic characteristics, the hydrophobic effect thereof is not enough, for example, a contact angle is less than 120 °. If step S200 is removed and thus there is no priority space occupation of the first non-polar medium 31, the first polar medium 32 will have a small convex curvature on a liquid surface toward the second non-polar medium 33 due to its insufficient hydrophobicity, resulting in that the formed membrane layers 30 have relatively large areas, and large and uneven thickness, and thus may lose functionality easily.

However, the first non-polar medium 31 of the embodiment of the present application is precoated in the pore array layered structure 10 and occupies spaces in the pore array layered structure 10 prior to the first polar medium 32, which can ensure relative insulation among the respective pore units 13 of the pore array layered structure 10 and avoid crosstalk of electrical signals. Moreover, the priority space occupation of the first non-polar medium 31 facilitates to generate a large convex curvature of the liquid surface of the first polar medium 32 toward the second non-polar medium 33, and the formed membrane layers 30 have uniform, stable and thin thickness.

According to the membrane forming method of the embodiments of the present application, the first non-polar medium 31 forms the precoating layer on the inner surfaces of the pore array layered structure, which can guide the flow of the membrane layers 30 between adjacent pore units 13 and ensure the stability of the membrane layers 30.

In a fourth aspect, the embodiments of the present application provide a nanopore sequencing device, which includes the pore array layered structure 10 of the embodiments in the first aspect of the present application, the chip device of the embodiments in the second aspect of the present application, or the membrane layers 30 prepared by the membrane forming method of the embodiments in the third aspect of the present application.

The fifth aspect of the present application relates to use of the pore array layered structure 10 of the embodiments in the first aspect of the present application, the chip device of the embodiments in the second aspect of the present application, or the membrane layers 30 prepared by the membrane forming method of the embodiments in the third aspect of the present application in characterizing an analyte, wherein the analyte includes biological polymer, and the biological polymer is selected from one of polynucleotide, polypeptide, polyose and lipide.

The embodiments in the fifth aspect of the present application provide a method for characterizing an analyte, including sequencing the analyte by use of the pore array layered structure 10 of the embodiments in the first aspect of the present application, the chip device of the embodiments in the second aspect of the present application, or the membrane layers 30 prepared by the membrane forming method of the embodiments in the third aspect of the present application, wherein the analyte includes biopolymer, and the biological polymer is selected from at least one of polynucleotide, polypeptide, polyose and lipide. An example of the biopolymer is polynucleotide, which includes DNA and/or RNA.

Although the present application has been described with reference to the preferred embodiments, without departing from the scope of the present application, various improvements can be made and components can be replaced with equivalents. In particular, as long as there is no structural conflict, the various technical features mentioned in various embodiments can be combined in any manner. The present application is not limited to the specific embodiments disclosed herein, but includes all technical solutions falling within the scope of the claims.

## Claims

1. A pore array layered structure for forming a membrane-forming space with a substrate, wherein the membrane-forming space is adapted to form a membrane layer, and the pore array layered structure comprises: a plurality of pore units arranged in an array, the pore units penetrating through the pore array layered structure and each comprising a first containing cavity and a second containing cavity communicated in an axial direction, wherein the first containing cavities are connected with the substrate, and at least one of the first containing cavities is communicated with other first containing cavities.

2. The pore array layered structure according to claim 1, wherein at least one of the second containing cavities is communicated with other second containing cavities.

3. The pore array layered structure according to claim 1 or 2, wherein the pore array layered structure comprises a first defining layer and a second defining layer, the first containing cavities are disposed in the first defining layer and penetrate through the first defining layer, and the second containing cavities are disposed in the second defining layer and penetrate through the second defining layer.

4. The pore array layered structure according to claim 3, wherein the second defining layer comprises a plurality of second sub units arranged in an array, and the second containing cavities are disposed in the second sub units and penetrate through the second sub units.

5. The pore array layered structure according to claim 4, wherein the second defining layer further comprises a plurality of third sub units arranged in an array, the third sub units comprise third sub containing cavities, and the second sub units are located in the third sub containing cavities with gaps being formed between the second sub units and the third sub units; and
optionally, a plurality of first grooves are provided on outer peripheral sides of respective second sub units.

6. The pore array layered structure according to claim 3, wherein the first defining layer comprises at least one first channel, by which at least two adjacent first containing cavities are communicated with each other; and/or
the second defining layer comprises at least one second channel, by which at least two adjacent second containing cavities are communicated with each other.

7. The pore array layered structure according to claim 3, wherein the first defining layer comprises at least one first communicating groove, the first communicating groove extends outward from the first containing cavity in a radial direction, and an end of the first communicating groove facing away from the first containing cavity is closed; optionally, the first communicating groove penetrates through the first defining layer; and/or
the second defining layer comprises at least one second communicating groove, the second communicating groove extends outward from the second containing cavity in a radial direction, and an end of the second communicating groove facing away from the second containing cavity is closed; optionally, the second communicating groove penetrates through the second defining layer.

8. The pore array layered structure according to claim 7, wherein the first defining layer comprises at least one first communicating groove, the at least one first communicating groove forms a third containing cavity, and the third containing cavity has a diameter or width greater than that of the second containing cavity; optionally, the first containing cavity has a diameter or width less than or equal to that of the second containing cavity.

9. A chip device, comprising
a substrate;
the pore array layered structure according to claim 1, wherein the pore array layered structure is located on the substrate, the pore array layered structure comprises a plurality of pore units arranged in an array, and second containing cavities of the pore units are located on the side of the first containing cavities of the pore units opposite to the substrate; and
membrane layers located in the plurality of pore units, wherein the membrane layers are configured to test samples to be tested.

10. A membrane forming method, comprising:
providing the chip device according to claim 9, wherein the chip device comprises the pore array layered structure;
disposing a first non-polar medium in the pore array layered structure and forming a precoating layer on a surface of the pore array layered structure;
flowing a first polar medium through the pore array layered structure to replace at least part of the first non-polar medium;
flowing a second non-polar medium through the pore array layered structure to replace at least part of the first polar medium, wherein the second non-polar medium comprises amphiphilic molecular materials; and
flowing a second polar medium through the pore array layered structure to replace at least part of the second non-polar medium and to form membrane layers at interface between the first polar medium and the second polar medium, wherein the membrane layers contain the amphiphilic molecular materials.

11. A nanopore sequencing device, comprising the pore array layered structure according to any of claims 1 to 8, the chip device according to claim 9, or the membrane layers prepared by the membrane forming method according to claim 10.

12. A use of the pore array layered structure according to any of claims 1 to 8, the chip device according to claim 9 or the membrane layers prepared by the membrane forming method according to claim 10 in characterizing an analyte, wherein the analyte comprises biological polymer, and the biological polymer is selected from one of polynucleotide, polypeptide, polyose and lipide, preferably the polynucleotide, which comprises DNA and/or RNA.
